# EUROPEAN PATENT SPECIFICATION

(11) **EP 1 771 465 B1**
(45) Date of publication and mention of the grant of the patent: **11.07.2012**
(21) Application number: 05762788.7
(22) Date of filing: 22.07.2005
(51) Int. Cl.: C12N 15/10

(54) **CELL CYCLE REPORTING CELL LINE**
ZELLINIE, DIE ÜBER DEN STATUS DES ZELLZYKLUS AUSKUNFT GIBT
LIGNÉE CELLULAIRE QUI REVÈLE L'ÉTAT DU CYCLE CELLULAIRE

(30) Priority: 23.07.2004 US 590814 P; 21.01.2005 US 645915 P; 21.01.2005 US 645968 P
(43) Date of publication of application: 11.04.2007
(73) Proprietor: GE Healthcare UK Limited, Buckinghamshire HP7 9NA (GB); Vanderbilt University, Nashville, TN 37235 (US)
(72) Inventor: HANCOCK, Suzanne, The Maynard Centre, Whitchurch, Cardiff CF14 7YT (GB); STUBBS, Simon, Whitchurch, Cardiff CF14 7YT (GB); THOMAS, Nicholas, Whitchurch, Cardiff CF14 7YT (GB); FANNING, Ellen, Dep. of B.&S., Vanderbilt Uni., Nashville, Tennessee 37235-1634 (US); GU, Jinming, Boston, Massachusetts 02115 (US)
(74) Representative: Bryan, Ian Bennett
(86) International application number: PCT/GB2005/002890
(87) International publication number: WO 2006/008547

(56) References cited:
- GU JINMING ET AL: "Cell cycle-dependent regulation of a human DNA helicase that localizes in DNA damage foci" MOLECULAR BIOLOGY OF THE CELL, vol. 15, no. 7, July 2004 (2004-07), pages 3320-3332, XP002353578 ISSN: 1059-1524 cited in the application
- FILHOL ODILE ET AL: "Live-cell fluorescence imaging reveals the dynamics of protein kinase CK2 individual subunits." MOLECULAR AND CELLULAR BIOLOGY, vol. 23, no. 3, February 2003 (2003-02), pages 975-987, XP002353579 ISSN: 0270-7306
- OAKLEY R H ET AL: "THE CELLULAR DISTRIBUTION OF FLUORESCENTLY LABELED ARRESTINS PROVIDES A ROBUST, SENSITIVE, AND UNIVERSAL ASSAY FOR SCREENING G PROTEIN-COUPLED RECEPTORS" ASSAY AND DRUG DEVELOPMENT TECHNOLOGIES, MARY ANN LIEBERT, NEW YORK, NY, US, vol. 1, no. 1-1, November 2002 (2002-11), pages 21-30, XP001181634 ISSN: 1540-658X

## Description

### Technical Field

The present invention relates to novel non-destructive and dynamic means for determining the cell cycle position of living cells.

### Background of the Invention

Eukaryotic cell division proceeds through a highly regulated cell cycle comprising consecutive phases termed G1, S, G2 and M. The two transition phases, G1 and G2, are interspersed between the DNA synthesis (S) phase in which cellular DNA is replicated and the mitosis (M) phase in which each cell divides to form two daughter cells.

Disruption of the cell cycle or cell cycle control can result in cellular abnormalities or disease states such as cancer which arise from multiple genetic changes that transform growth-limited cells into highly invasive cells that are unresponsive to normal control of growth. Transition of normal cells into cancer cells arises though loss of correct function in DNA replication and DNA repair mechanisms. All dividing cells are subject to a number of control mechanisms, known as cell-cycle checkpoints, which maintain genomic integrity by arresting or inducing destruction of aberrant cells. Investigation of cell cycle progression and control is consequently of significant interest in designing anticancer drugs (Flatt PM and Pietenpol JA 2000 Drug Metab Rev 32(3-4):283-305; Buolamwini JK 2000 Current Pharmaceutical Design 6, 379-392).

Accurate determination of cell cycle status is a key requirement for investigation of cellular processes which affect the cell cycle or are dependent on cell cycle position. Such measurements are particularly vital in drug screening applications where:
a) drugs which directly or indirectly modify cell cycle progression are desired, for example as anti-cancer treatments.
b) drugs are to be checked for unwanted effects on cell cycle progression.
c) it is suspected that an agent is active or inactive towards cells in a particular phase of the cell cycle.

Traditionally cell cycle status for cell populations has been determined by flow cytometry using fluorescent dyes which stain the DNA content of cell nuclei (Barlogie B etal. Cancer Res. 1983 43(9):3982-97). Flow cytometry yields quantitative information on the DNA content of cells and hence allows determination of the relative numbers of cells in, or the proportion of cells in,the G1, S and G2+M phases of the cell cycle. However this analysis is a destructive non-dynamic process and requires serial sampling of a population to determine cell cycle status with time. Furthermore standard flow cytometry techniques examine only the total cell population in the sample and do not yield data on individual cells which precludes study of cell cycle status of different cell types that may be present within the sample under analysis. Flow cytometry is therefore suitable for examining the overall cell cycle distribution of cells within a population but cannot be used to monitor the precise cell cycle status of an individual cell over time.

Consequently what is needed to study the effects of agents with desired or undesired effects on the cell cycle is a method to precisely determine cell cycle status of a single living cell by a non-destructive method that allows the same cell to be repeatedly interrogated over time. Furthermore it would be advantageous for cell cycle position to be determined from a probe controlled directly by cell cycle control components, rather than indirectly through DNA content or other indirect markers of cell cycle position as described above.

A number of methods have been described which make use of certain components of the cell cycle control mechanisms to provide procedures which analyse or exploit cell proliferation status.

US 6048693 describes a method for screening for compounds affecting cell cycle regulatory proteins wherein expression of a reporter gene is linked to control elements which are acted on by cyclins or other cell cycle control proteins. In this method temporal expression of a reporter gene product is driven in a cell cycle specific fashion and compounds acting on one or more cell cycle control components may increase or decrease expression levels. Since the assay system contains no elements which provide for the destruction of the reporter gene product nor for destruction of any signal arising from the reporter gene, the method cannot yield information on the cell cycle position of any cells in the assay and reports only on general perturbations of cell cycle control mechanisms.

WO 03/031612 describes DNA reporter constructs and methods for determining the cell cycle position of living mammalian cells by means of cell cycle phase-specific expression control elements and destruction control elements. One embodiment uses well characterised elements of the cell cycle control protein Cyclin B1 to control the expression and degradation of a green fluorescent protein (GFP) molecule to report cellular transition through G2 and M phases of the cell cycle. Since this construct is under the control of the Cyclin B1 promoter, GFP expression is absent during G1 and S thus preventing analysis of cells in these phases of the cell cycle.

A human helicase B homolog has been reported and characterised ((Taneja et al J. Biol. Chem., (2002), 277, 40853-40861). The report demonstrates that helicase activity is needed during G1 to promote the G1/S transition.

Gu et al (Mol. Biol. Cell., (2004), 15, 3320-3332) have shown that a small C-terminal region of the helicase B gene termed the phosphorylation-dependent subcellular localization domain (PSLD) is phosphorylated by Cdk2/cyclin E and contains NLS and NES sequences. Gu et al (Mol. Biol. Cell., (2004), 15, 3320-3332) carried out studies on cells that had been transiently transfected with plasmid encoding an EGFP-βGal-PSLD fusion (beta-galactosidase (βGal) was included in the construct as an inert group to make the whole fusion protein similar in size to the complete helicase B) expressed from a CMV promoter. Cells in G1 exhibited EGFP signal predominantly in the nucleus, whilst cells in other phases of the cell cycle exhibited predominantly cytoplasmic EGFP signal. These researchers concluded that the PSLD was directing translocation of the βGal-EGFP reporter from the nucleus to the cytoplasm around the G1/S phase transition of the cell cycle.

None of the preceding methods which use components of the cell cycle control mechanism provides means for readily and accurately determining the cell cycle status of an individual cell or a population of cells throughout the entire cell cycle. Accordingly a method has been developed and is herein described which uses key components of the cell cycle regulatory machinery, in defined combinations, to drive dual independent cellular reporters to provide novel means of determining cell cycle status at all phases of the cell cycle in individual living cells.

### Summary of the Invention

According to the first aspect of the invention, there is provided a stable cell line expressing:
i) a first polypeptide construct comprising a first fluorescent protein linked to a phosphorylation-dependent subcellular localization domain of the C-terminal special control region of helicase B (PSLD), the location of which construct within a mammalian cell is indicative of the cell cycle position; and
ii) a second polypeptide construct comprising a second fluorescent protein linked to a Cyclin B1 D-box wherein said second fluorescent protein is detectable in a mammalian cell at a predetermined position in the cell cycle,
wherein said first and second fluorescent proteins are distinguishable from each other and the stable cell line can be used to determine the cell cycle position.

The present invention provides cell lines containing polypeptide constructs which exhibit cell cycle phase specific activation, translocation or destruction of detectable reporter molecules by direct linkage of reporter signals to temporal and spatial expression, localisation and destruction of cell cycle components. This greatly improves the precision of determination of cell cycle phase status and allows continuous monitoring of cell cycle progression in individual cells. Furthermore the inventors have discovered that these key control elements can be isolated and abstracted from functional elements of the cell cycle control mechanism to permit design of cell cycle phase reporters which are dynamically regulated and operate in concert with, but independently of, endogenous cell cycle control components and hence provide means for monitoring cell cycle status without influencing or interfering with the natural progression of the cell cycle.

Preferably, said fluorescent protein is selected from the group consisting of Green Fluorescent Protein (GFP), Enhanced Green Fluorescent Protein (EGFP), Emerald and J-Red.

Preferably, the first fluorescent protein is EGFP and the second fluorescent protein is J-Red, or the first fluorescent protein is J-Red and the second fluorescent protein is EGFP. More preferably, the first fluorescent protein is J-Red and the second fluorescent protein is EGFP.

In one preferred embodiment of the invention the fluorescent protein of the first polypeptide construct is a red fluorescent protein (RFP), and the second polypeptide construct comprises 171 amino acids of the amino terminus of cyclin B1 coupled to a green fluorescent protein (GFP) expressed under the control of the cyclin B1 promoter.

When expressed in a mammalian cell these constructs exhibit cell cycle specific expression and destruction of the GFP construct and translocation of the RFP construct, with the GFP construct paralleling the expression and degradation of endogenous cyclin B1, and the RFP construct paralleling the translocation of endogenous Helicase B. Hence measurement of both GFP and RFP fluorescence intensity and localisation permits identification of cells in G1, S, G2 and M phases of the cell cycle. Analysis of the fluorescence characteristics of each cell in a population with time consequently yields dynamic information on the cell cycle status of each cell.

In further aspects of the invention there are provided methods for analysing cell cycle distribution of cultured cells and determining the effects of test agents on cell cycle distribution. The term 'test agent' should be construed as a form of electromagnetic radiation or as a chemical entity. Preferably, the test agent is a chemical entity selected from the group consisting of drug, nucleic acid, hormone, protein and peptide. The test agent may be applied exogenously to the cell or may be a peptide or protein that is expressed in the cell under study.

Thus, in a second aspect of the present invention, there is provided a method for determining the cell cycle position of a mammalian cell, said method comprising:
a) culturing a stable cell line as hereinbefore described; and
b) determining the cell cycle position by monitoring signals emitted by the first and second fluorescent proteins.

In a third aspect of the present invention, there is provided a method of determining the effect of a test agent on the cell cycle position of a mammalian cell, said method comprising:
a) culturing a stable cell line as hereinbefore described; and
b) determining the cell cycle position by monitoring signals emitted by the first and second fluorescent proteins wherein a difference between the emitted signals measured in the absence and in the presence of said test agent is indicative of the effect of the test agent on the cell cycle position of the cell.

In a fourth aspect of the present invention, there is provided a method of determining the effect of a test agent on the cell cycle position of a mammalian cell, said method comprising:
a) culturing a stable cell line as hereinbefore described;
b) determining the cell cycle position by monitoring signals emitted by the first and second fluorescent proteins; and
c) comparing the emitted signals in the presence of the test agent with a known value for the emitted signals in the absence of the test agent;
wherein a difference between the emitted signals measured in the presence of the test agent and said known value in the absence of the test agent is indicative of the effect of the test agent on the cell cycle position of the cell.

In yet a further aspect of the present invention methods are provided for determining the cell cycle dependencies of cellular processes by means of monitoring cellular processes in cells reporting the cell cycle position.

Thus, according to the fifth aspect of the present invention, there is provided a method of determining the effect of the mammalian cell cycle on a cellular process monitored by a process reporter which is known to vary in response to a test agent, said method comprising:
a) culturing a stable cell line as hereinbefore described;
b) determining the cell cycle position by monitoring signals emitted by the first and second fluorescent proteins; and
c) monitoring the signals emitted by the process reporter wherein the process reporter is distinguishable from the first and second fluorescent proteins;
wherein the relationship between cell cycle position determined by step b) and the signal emitted by the process reporter is indicative of whether or not the cellular process is cell cycle dependent.

### Brief Description of the Drawings

SEQ ID NO: 1 is the amino acid sequence of the PSLD - J-RED fusion protein.
SEQ ID NO: 2 is the nucleic acid sequence for pCORON1022-JRed-C1-PSLD.
SEQ ID NO: 3 is the nucleic acid sequence for pCORON1020-JRed-C1-PSLD

Figure 1 is a vector map of pCORON1002-EGFP-C1-PSLD.
Figure 2 shows vector maps of pCORON1022-JRed-C1-PSLD (Figure 2a) and pCORON1020-JRed-C1-PSLD (Figure 2b).
Figure 3 presents images of U2OS cells expressing the cyclin B1-EGFP and the PSLD-J Red fluorescent proteins using an IN Cell Analyzer 1000 (GE Healthcare) imaging system. The cells are seen to be at varying stages of their cell cycle, as depicted by letters/numerals 'S', 'G1' and 'G2'. The presence of the Hoechst dye is indicated by the blue fluorescence in Figure 3a, of expression of the G2/M Cyclin B1 green fluorescent protein reporter in Figure 3b and the expression of the G1/S PSLD red fluorescent protein reporter in Figure 3c.

### Detailed Description of the Invention

### PSLD-RFP Construct

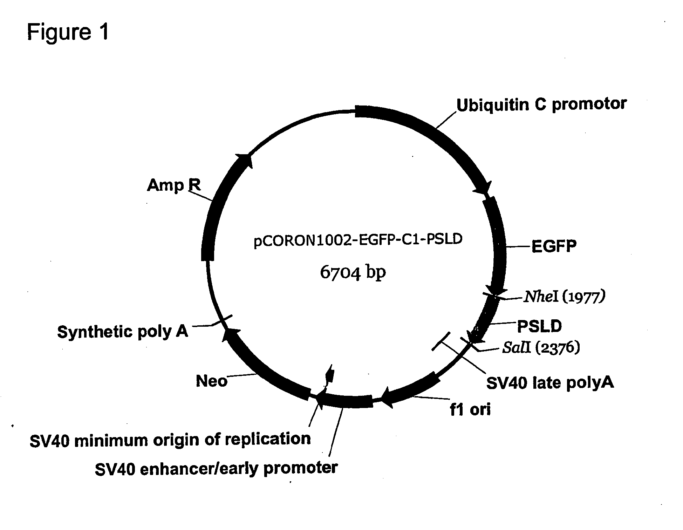

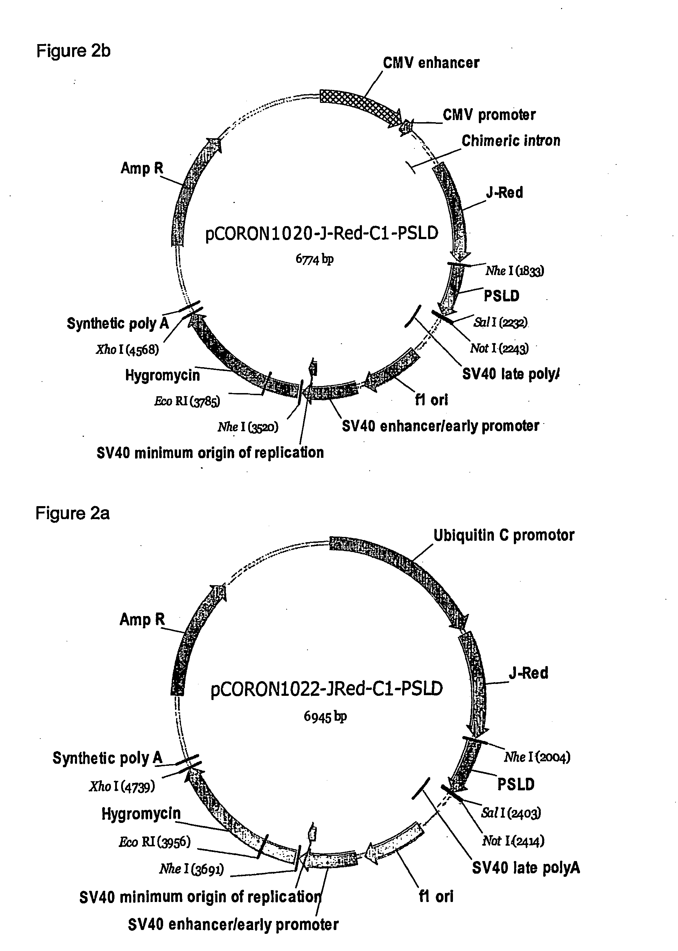

Full-length Human DNA helicase B (HDHB) cDNA was inserted as a BglII/Notl fragment (Taneja et al., J. Biol. Chem., (2002) 277, 40853-40861) into the Notl site of the pEGFP-C1 vector (Clontech). PCR amplification of the 390 bp PSLD region and introduction of 5' Nhel and 3' Sall restriction enzyme sites to the PSLD fragment were used to sub-clone into the vector pCORON1002-EGFP-C1 (GE Healthcare). The resulting 6704 bp DNA construct pCORON1002-EGFP-C1-PSLD (Figure 1), contains an ubiquitin C promoter, a bacterial ampicillin resistance gene and a mammalian neomycin resistance gene. Further modification of this vector was carried out using standard PCR and cloning techiques (Sambrook, J. et al (1989)) to replace the EGFP with the fluorescent protein J-Red (Evrogen), to convert the plasmid from the neomycin resistance to hygromycin resistance (Figure 2a) and to replace the ubiquitin C promoter with CMV IE/promoter (Figure 2b).

### Dual Construct Stable Cell Line

A U2OS cell line stably expressing a Cyclin B1-EGFP cell cycle reporter (as described in WO03/031612 and supplied under product code 25-80-10 'G2M Cell Cycle Phase Marker' from Amersham Biosciences UK Limited/GE Healthcare Biosciences) was cultured according to the supplier's instructions. Cells were transfected with plasmids (Figures 2a and 2b) encoding the PSLD-RFP fusion protein (SEQ ID NO: 1) using Fugene (Roche) according to the manufacturer's instructions. Cells were placed under hygromycin (125 µg/ml) and neomycin (500 µg/ml) selection, and surviving clones selected for further expansion.

### Imaging of stable cell line expressing G1/S and G2/M sensors

A stable U2OS cell line expressing Cyclin B1-EGFP and PSLD-J Red fluorescent fusion proteins was grown in 96 well plates in McCoys medium supplemented with 10% serum under standard tissue culture conditions. Cells were fixed in 2% paraformaldehyde, stained with Hoechst, and imaged using an IN Cell Analyzer 1000 (GE Healthcare) with appropriate excitation and emission filters for blue (Hoechst), green (Cyclin B1-EGFP) and red (PSLD-J Red) fluorescence.

### Example 1

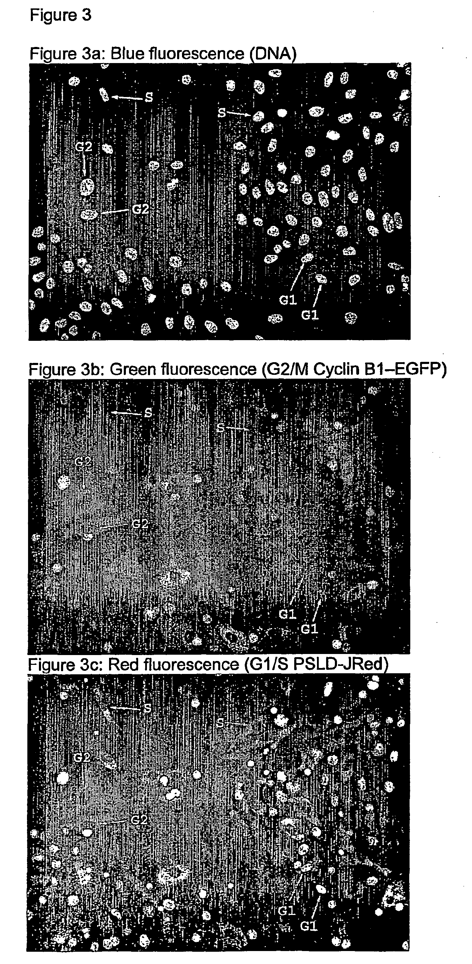

Images of the stable cell line cell line expressing Cyclin B1-EGFP and PSLD-J Red (Figure 3) show differential expression and localisation of the green and red fusion proteins between cells in different phases of the cell cycle. Determination of the presence or absence of green and red fluorescent fusion proteins in the cytoplasm and nucleus of each cell allowed designation of cell cycle position according to the following scheme:

| | | G1 | S | G2 | M |
|---|---|---|---|---|---|
| Cytoplasm | Green | - | - | + | + |
| | Red | - | + | + | + |
| Nucleus | Green | - | - | - | + |
| | Red | + | - | - | + |

Experimental details relating to the production of stable cell lines expressing a polypeptide construct comprising a first detectable live-cell reporter molecule linked to at least one cell cycle phase-dependent location control element, the location of which construct within a mammalian cell is indicative of the cell cycle position, have been described in Applicant's copending US provisional patent application US60/645,968 entitled "Cell Cycle Phase Markers" .

The foregoing is illustrative of the present invention and is not to be construed as limiting thereof. Although a few exemplary embodiments of this invention have been described, those skilled in the art will readily appreciate that many modifications are possible in the exemplary embodiments without materially departing from the novel teachings and advantages of this invention. Accordingly, all such modifications are intended to be included within the scope of this invention as defined in the claims. Therefore, it is to be understood that the foregoing is illustrative of the present invention and is not to be construed as limited to the specific embodiments disclosed, and that modifications to the disclosed embodiments, as well as other embodiments, are intended to be included within the scope of the appended claims. The invention is defined by the following claims.

### SEQUENCE LISTING

<110> Amersham Biosciences UK Limited
<120> Cell Cycle Reporting Cell Line
<130> PA0503-PCT
<140> PCT/GB2005/002890
   <141> 2005-07-22
<150> US 60/590814
   <151> 2004-07-23
<150> US 60/645915
   <151> 2005-01-21
<150> US 60/645968
   <151> 2005-01-21
<160> 3
<170> PatentIn version 3.3
<210> 1
   <211> 385
   <212> PRT
   <213> Artificial
<220>
   <223> PSLD- J-RED fusion protein
<400> 1
<210> 2
   <211> 6945
   <212> DNA
   <213> Artificial
<220>
   <223> PCORON1022-JRed-C1-PSLD
<400> 2
   ggcctccgcg ccgggttttg gcgcctcccg cgggcgcccc cctcctcacg gcgagcgctg      60
   ccacgtcaga cgaagggcgc agcgagcgtc ctgatccttc cgcccggacg ctcaggacag     120
   cggcccgctg ctcataagac tcggccttag aaccccagta tcagcagaag gacattttag     180
   gacgggactt gggtgactct agggcactgg ttttctttcc agagagcgga acaggcgagg     240
   aaaagtagtc ccttctcggc gattctgcgg agggatctcc gtggggcggt gaacgccgat     300
   gattatataa ggacgcgccg ggtgtggcac agctagttcc gtcgcagccg ggatttgggt 360
   cgcggttctt gtttgtggat cgctgtgatc gtcacttggt gagtagcggg ctgctgggct 420
   ggccggggct ttcgtggccg ccgggccgct cggtgggacg gaagcgtgtg gagagaccgc 480
   caagggctgt agtctgggtc cgcgagcaag gttgccctga actgggggtt ggggggagcg 540
   cagcaaaatg gcggctgttc ccgagtcttg aatggaagac gcttgtgagg cgggctgtga 600
   ggtcgttgaa acaaggtggg gggcatggtg ggcggcaaga acccaaggtc ttgaggcctt 660
   cgctaatgcg ggaaagctct tattcgggtg agatgggctg gggcaccatc tggggaccct 720
   gacgtgaagt ttgtcactga ctggagaact cggtttgtcg tctgttgcgg gggcggcagt 780
   tatggcggtg ccgttgggca gtgcacccgt acctttggga gcgcgcgccc tcgtcgtgtc 840
   gtgacgtcac ccgttctgtt ggcttataat gcagggtggg gccacctgcc ggtaggtgtg 900
   cggtaggctt ttctccgtcg caggacgcag ggttcgggcc tagggtaggc tctcctgaat 960
   cgacaggcgc cggacctctg gtgaggggag ggataagtga ggcgtcagtt tctttggtcg 1020
   gttttatgta cctatcttct taagtagctg aagctccggt tttgaactat gcgctcgggg 1080
   ttggcgagtg tgttttgtga agttttttag gcaccttttg aaatgtaatc atttgggtca 1140
   atatgtaatt ttcagtgtta gactagtaaa ttgtccgcta aattctggcc gtttttggct 1200
   tttttgttag acgaagcttg gtaccgagct cgatatcgcc accatggacg aggatggttc 1260
   agagggcggc cccgccctgt tccagagcga catgaccttc aaaatcttca tcgacggcga 1320
   ggtgaacggc cagaagttca ccatcgtggc cgacggcagc agcaagttcc cccacggcga 1380
   cttcaacgtg cacgccgtgt gcgagaccgg caagctgccc atgagctgga agcccatctg 1440
   ccacctgatc cagtacggcg agcccttctt cgcccgctac cccaacggca tcagccactt 1500
   cgcccaggag tgcttccccg agggcctgag catcgaccgc accgtgcgct tcgagaacga 1560
   cggcaccatg accagccacc acacctacga gctggacggc acctgcgtgg tcagccgcat 1620
   caccgtgaac tgcgacggct tccagcccga cggccccatc atgcgcgacc agctggtgga 1680
   catcctgccc aacgagaccc acatgttccc ccacggcccc aacgccgtgc gccagctggc 1740
   cttcatcggc ttcaccaccg ccgacggcgg cctgatgatg ggccacttcg acagcaagat 1800
   gaccttcaac ggcagccgcg ccatcaagat ccccggcccc cacttcgtga ccatcatcac 1860
   caagcagatg agggacacca gcgacaagcg cgaccacgtg tgccagcgcg aggtgaccta 1920
   cgcccacagc gtgccccgca tcaccagcgc catcggtagc gacgaggatt ccggactcat 1980
   gtacaagggc aatggcggca atgctagcag cggcgcacct ccagcagatt ttccgtcccc 2040
   acggaagagc tctggagaca gtggaggacc cagcacaccg tcagcatctc cactccctgt 2100
   agtcacagac cacgccatga caaatgatgt cacctggagc gaggcctctt cgcctgatga 2160
   gaggacactc acctttgctg aaagatggca attatcttca cctgatggag tagatacaga 2220
   tgatgattta ccaaaatcgc gagcatccaa aagaacctgt ggtgtgaatg atgatgaaag 2280
   tccaagcaaa atttttatgg tgggagaatc tccacaagtg tcttccagac ttcagaattt 2340
   gagactgaat aatttaattc ccaggcaact tttcaagccc accgataatc aagaaactta 2400
   ggtcgacccg ggcggccgct tcgagcagac atgataagat acattgatga gtttggacaa 2460
   accacaacta gaatgcagtg aaaaaaatgc tttatttgtg aaatttgtga tgctattgct 2520
   ttatttgtaa ccattataag ctgcaataaa caagttaaca acaacaattg cattcatttt 2580
   atgtttcagg ttcaggggga gatgtgggag gttttttaaa gcaagtaaaa cctctacaaa 2640
   tgtggtaaaa tccgataagg atcgatccgg gctggcgtaa tagcgaagag gcccgcaccg 2700
   atcgcccttc ccaacagttg cgcagcctga atggcgaatg gacgcgccct gtagcggcgc 2760
   attaagcgcg gcgggtgtgg tggttacgcg cagcgtgacc gctacacttg ccagcgccct 2820
   agcgcccgct cctttcgctt tcttcccttc ctttctcgcc acgttcgccg gctttccccg 2880
   tcaagctcta aatcgggggc tccctttagg gttccgattt agtgctttac ggcacctcga 2940
   ccccaaaaaa cttgattagg gtgatggttc acgtagtggg ccatcgccct gatagacggt 3000
   ttttcgccct ttgacgttgg agtccacgtt ctttaatagt ggactcttgt tccaaactgg 3060
   aacaacactc aaccctatct cggtctattc ttttgattta taagggattt tgccgatttc 3120
   ggcctattgg ttaaaaaatg agctgattta acaaaaattt aacgcgaatt ttaacaaaat 3180
   attaacgctt acaatttcct gatgcggtat tttctcctta cgcatctgtg cggtatttca 3240
   caccgcatac gcggatctgc gcagcaccat ggcctgaaat aacctctgaa agaggaactt 3300
   ggttaggtac cttctgaggc ggaaagaacc agctgtggaa tgtgtgtcag ttagggtgtg 3360
   gaaagtcccc aggctcccca gcaggcagaa gtatgcaaag catgcatctc aattagtcag 3420
   caaccaggtg tggaaagtcc ccaggctccc cagcaggcag aagtatgcaa agcatgcatc 3480
   tcaattagtc agcaaccata gtcccgcccc taactccgcc catcccgccc ctaactccgc 3540
   ccagttccgc ccattctccg ccccatggct gactaatttt ttttatttat gcagaggccg 3600
   aggccgcctc ggcctctgag ctattccaga agtagtgagg aggctttttt ggaggcctag 3660
   gcttttgcaa aaagcttgat tcttctgacg ctagcgatcg cccgggccac catgaaaaag 3720
   cctgaactca ccgcgacgtc tgtcgagaag tttctgatcg aaaagttcga cagcgtctcc 3780
   gacctgatgc agctctcgga gggcgaagaa tctcgtgctt tcagcttcga tgtaggaggg 3840
   cgtggatatg tcctgcgggt aaatagctgc gccgatggtt tctacaaaga tcgttatgtt 3900
   tatcggcact ttgcatcggc cgcgctcccg attccggaag tgcttgacat tggggaattc 3960
   agcgagagcc tgacctattg catctcccgc cgtgcacagg gtgtcacgtt gcaagacctg 4020
   cctgaaaccg aactgcccgc tgttctgcag ccggtcgcgg aggccatgga tgcgatcgct 4080
   gcggccgatc ttagccagac gagcgggttc ggcccattcg gaccgcaagg aatcggtcaa 4140
   tacactacat ggcgtgattt catatgcgcg attgctgatc cccatgtgta tcactggcaa 4200
   actgtgatgg acgacaccgt cagtgcgtcc gtcgcgcagg ctctcgatga gctgatgctt 4260
   tgggccgagg actgccccga agtccggcac ctcgtgcacg cggatttcgg ctccaacaat 4320
   gtcctgacgg acaatggccg cataacagcg gtcattgact ggagcgaggc gatgttcggg 4380
   gattcccaat acgaggtcgc caacatcttc ttctggaggc cgtggttggc ttgtatggag 4440
   cagcagacgc gctacttcga gcggaggcat ccggagcttg caggatcgcc gcggctccgg 4500
   gcgtatatgc tccgcattgg tcttgaccaa ctctatcaga gcttggttga cggcaatttc 4560
   gatgatgcag cttgggcgca gggtcgatgc gacgcaatcg tccgatccgg agccgggact 4620
   gtcgggcgta cacaaatcgc ccgcagaagc gcggccgtct ggaccgatgg ctgtgtagaa 4680
   gtactcgccg atagtggaaa ccgacgcccc agcactcgtc cgagggcaaa ggaatagctc 4740
   gagtttcgaa atgaccgacc aagcgacgcc caacctgcca tcacgatggc cgcaataaaa 4800
   tatctttatt ttcattacat ctgtgtgttg gttttttgtg tgaatcgata gcgataagga 4860
   tccgcgtatg gtgcactctc agtacaatct gctctgatgc cgcatagtta agccagcccc 4920
   gacacccgcc aacacccgct gacgcgccct gacgggcttg tctgctcccg gcatccgctt 4980
   acagacaagc tgtgaccgtc tccgggagct gcatgtgtca gaggttttca ccgtcatcac 5040
   cgaaacgcgc gagacgaaag ggcctcgtga tacgcctatt tttataggtt aatgtcatga 5100
   taataatggt ttcttagacg tcaggtggca cttttcgggg aaatgtgcgc ggaaccccta 5160
   tttgtttatt tttctaaata cattcaaata tgtatccgct catgagacaa taaccctgat 5220
   aaatgcttca ataatattga aaaaggaaga gtatgagtat tcaacatttc cgtgtcgccc 5280
   ttattccctt ttttgcggca ttttgccttc ctgtttttgc tcacccagaa acgctggtga 5340
   aagtaaaaga tgctgaagat cagttgggtg cacgagtggg ttacatcgaa ctggatctca 5400
   acagcggtaa gatccttgag agttttcgcc ccgaagaacg ttttccaatg atgagcactt 5460
   ttaaagttct gctatgtggc gcggtattat cccgtattga cgccgggcaa gagcaactcg 5520
   gtcgccgcat acactattct cagaatgact tggttgagta ctcaccagtc acagaaaagc 5580
   atcttacgga tggcatgaca gtaagagaat tatgcagtgc tgccataacc atgagtgata 5640
   acactgcggc caacttactt ctgacaacga tcggaggacc gaaggagcta accgcttttt 5700
   tgcacaacat gggggatcat gtaactcgcc ttgatcgttg ggaaccggag ctgaatgaag 5760
   ccataccaaa cgacgagcgt gacaccacga tgcctgtagc aatggcaaca acgttgcgca 5820
   aactattaac tggcgaacta cttactctag cttcccggca acaattaata gactggatgg 5880
   aggcggataa agttgcagga ccacttctgc gctcggccct tccggctggc tggtttattg 5940
   ctgataaatc tggagccggt gagcgtgggt ctcgcggtat cattgcagca ctggggccag 6000
   atggtaagcc ctcccgtatc gtagttatct acacgacggg gagtcaggca actatggatg 6060
   aacgaaatag acagatcgct gagataggtg cctcactgat taagcattgg taactgtcag 6120
   accaagttta ctcatatata ctttagattg atttaaaact tcatttttaa tttaaaagga 6180
   tctaggtgaa gatccttttt gataatctca tgaccaaaat cccttaacgt gagttttcgt 6240
   tccactgagc gtcagacccc gtagaaaaga tcaaaggatc ttcttgagat cctttttttc 6300
   tgcgcgtaat ctgctgcttg caaacaaaaa aaccaccgct accagcggtg gtttgtttgc 6360
   cggatcaaga gctaccaact ctttttccga aggtaactgg cttcagcaga gcgcagatac 6420
   caaatactgt tcttctagtg tagccgtagt taggccacca cttcaagaac tctgtagcac 6480
   cgcctacata cctcgctctg ctaatcctgt taccagtggc tgctgccagt ggcgataagt 6540
   cgtgtcttac cgggttggac tcaagacgat agttaccgga taaggcgcag cggtcgggct 6600
   gaacgggggg ttcgtgcaca cagcccagct tggagcgaac gacctacacc gaactgagat 6660
   acctacagcg tgagctatga gaaagcgcca cgcttcccga agggagaaag gcggacaggt 6720
   atccggtaag cggcagggtc ggaacaggag agcgcacgag ggagcttcca gggggaaacg 6780
   cctggtatct ttatagtcct gtcgggtttc gccacctctg acttgagcgt cgatttttgt 6840
   gatgctcgtc aggggggcgg agcctatgga aaaacgccag caacgcggcc tttttacggt 6900
   tcctggcctt ttgctggcct tttgctcaca tggctcgaca gatct 6945
<210> 3
   <211> 6774
   <212> DNA
   <213> Artificial
<220>
   <223> pCORON1020-JRED-C1-PSLD
<400> 3

### organization Applicant

Street : GE Healthcare Limited, Amersham Place
   City : Little Chalfont
   State : Buckinghamshire
   Country : UK
   PostalCOde : HP7 9NA
   PhoneNumber : 44 1494 542290
   FaxNumber : 44 1494 542996
   EmailAddress : ian.bryan@ge.com
<110> OrganizationName : Amersham Biosciences UK Limited

### Application Project

<120> Title : Cell Cycle Reporting Cell Line
   <130> AppFileReference : PA0503-PCT
   <140> currentAppNumber : PCT/GB2005/002890
   <141> currentFilingDate : 2005-07-22

### Earlier Applications

<150> PriorAppNumber : US 60/590814
   <151> PriorFilingDate : 2004-07-23

### Earlier Applications

<150> PriorAppNumber : us 60/645915
   <151> PriorFilingDate : 2005-01-21

### Earlier Applications

<150> PriorAppNumber : US 60/645968
   <151> PriorFilingDate : 2005-01-21

### Sequence

<213> OrganismName : Artificial
   <400> PreSequenceString :
<212> Type : PRT
   <211> Length : 385
   SequenceName : SEQ ID NO: 1
   SequenceDescription :

### Sequence

<213> OrganismName : Artificial
   <400> Presequencestring :
<212> Type : DNA
   <211> Length : 6945
   SequenceName : SEQ ID NO: 2
   SequenceDescription :

### Sequence

<213> OrganismName : Artificial
   <400> PreSequenceString :
<212> Type : DNA
   <211> Length : 6774
   SequenceName : SEQ ID NO: 3
   SequenceDescription :

## Claims

1. A stable cell line expressing:
i) a first polypeptide construct comprising a first fluorescent protein linked to a phosphorylation-dependent subcellular localization domain of the C-terminal special control region of helicase B (PSLD), the location of which said construct within a mammalian cell is indicative of the cell cycle position; and
ii) a second polypeptide construct comprising a second fluorescent protein linked to a Cyclin B1 D-box wherein said second fluorescent protein is detectable in a mammalian cell at a predetermined position in the cell cycle,
wherein said first and second fluorescent proteins are distinguishable from each other and the stable cell line can be used to determine the cell cycle position.

2. The stable cell line of claim 1, wherein said fluorescent protein is selected from Green Fluorescent Protein (GFP), Enhanced Green Fluorescent Protein (EGFP), Emerald and J-Red.

3. A stable cell line according to any preceding claim wherein the first fluorescent protein is EGFP and the second fluorescent protein is J-Red, or the first fluorescent protein is J-Red and the second fluorescent protein is EGFP.

4. A stable cell line according to any preceding claim wherein the fluorescent protein of the first polypeptide construct is a red fluorescent protein (RFP), and the second polypeptide construct comprises 171 amino acids of the amino terminus of cyclin B1 coupled to a green fluorescent protein (GFP) expressed under the control of the cyclin B1 promoter.

5. A method for determining the cell cycle position of a mammalian cell, said method comprising:
a) culturing a stable cell line according to claims 1-4; and
b) determining the cell cycle position by monitoring signals emitted by the first and second fluorescent proteins.

6. A method for determining the effect of a test agent on the cell cycle position of a mammalian cell, said method comprising:
a) culturing a stable cell line according to claims 1-4; and
b) determining the cell cycle position by monitoring signals emitted by the first and second fluorescent proteins wherein a difference between the emitted signals measured in the absence and in the presence of said test agent is indicative of the effect of the test agent on the cell cycle position of the cell.

7. A method for determining the effect of a test agent on the cell cycle position of a mammalian cell, said method comprising:
a) culturing a stable cell line according to claims 1-4;
b) determining the cell cycle position by monitoring signals emitted by the first and second fluorescent proteins,
c) comparing the emitted signals in the presence of the test agent with a known value for the emitted signals in the absence of the test agent;
wherein a difference between the emitted signals measured in the presence of the test agent and said known value in the absence of the test agent is indicative of the effect of the test agent on the cell cycle position of the cell.

8. A method for determining the effect of the mammalian cell cycle on a cellular process monitored by a process reporter which is known to vary in response to a test agent, said method comprising:
a) culturing a stable cell line according to claims 1-4
b) determining the cell cycle position by monitoring signals emitted by the first and second fluorescent proteins; and
c) monitoring the signals emitted by the process reporter wherein the process reporter is distinguishable from the first and second fluorescent proteins,
wherein the relationship between cell cycle position determined by step b) and the signal emitted by the process reporter is indicative of whether or not the cellular process is cell cycle dependent.

## Patentansprüche

1. Stabile Zelllinie, die exprimiert:
i) ein erstes Polypeptidkonstrukt, umfassend ein erstes fluoreszierendes Protein, das mit einer phosphorylierungsabhängigen subzellulären Lokalisationsdomäne der C-terminalen speziellen Kontrollregion von Helikase B (PSLD) verknüpft ist, wobei der Ort des Konstrukts innerhalb einer Säugerzelle ein Indikator für die Zellzyklusposition ist; und
ii) ein zweites Polypeptidkonstrukt, umfassend ein zweites fluoreszierendes Protein, das mit einer Cyclin-B1-D-Box verknüpft ist, wobei das zweite fluoreszierende Protein in einer Säugerzelle an einer vorgegebenen Position im Zellzyklus nachweisbar ist,
wobei das erste und das zweite fluoreszierende Protein voneinander unterscheidbar sind und die stabile Zelllinie verwendet werden kann, um die Zellzyklusposition zu bestimmen.

2. Stabile Zelllinie nach Anspruch 1, wobei das fluoreszierende Protein aus grün fluoreszierendem Protein (GFP), verbessertem grün fluoreszierendem Protein (EGFP), Emerald und J-Red gewählt ist.

3. Stabile Zelllinie nach einem der vorstehenden Ansprüche, wobei das erste fluoreszierende Protein EGFP ist und das zweite fluoreszierende Protein J-Red ist, oder das erste fluoreszierende Protein J-Red ist und das zweite fluoreszierende Protein EGFP ist.

4. Stabile Zelllinie nach einem der vorstehenden Ansprüche, wobei das fluoreszierende Protein des ersten Polypeptidkonstrukts ein rot fluoreszierendes Protein (RFP) ist, und das zweite Polypeptidkonstrukt 171 Aminosäuren des Aminoterminus von Cyclin B1, gekoppelt an ein grün fluoreszierendes Protein (GFP), umfasst, das unter der Steuerung des Cyclin-B1-Promoters exprimiert wird.

5. Verfahren zur Bestimmung der Zellzyklusposition einer Säugerzelle, wobei das Verfahren umfasst:
a) Kultivieren einer stabilen Zelllinie nach Ansprüchen 1 - 4; und
b) Bestimmen der Zellzyklusposition durch Überwachen von Signalen, die vom ersten und zweiten fluoreszierenden Protein gesendet werden.

6. Verfahren zur Bestimmung der Wirkung eines Testmittels auf die Zellzyklusposition einer Säugerzelle, wobei das Verfahren umfasst:
a) Kultivieren einer stabilen Zelllinie nach Ansprüchen 1 - 4; und
b) Bestimmen der Zellzyklusposition durch Überwachen von Signalen, die vom ersten und zweiten fluoreszierenden Protein gesendet werden, wobei ein Unterschied zwischen den gesendeten Signalen, die bei Nichtvorhandensein und bei Vorhandensein des Testmittels gemessen werden, ein Indikator für die Wirkung des Testmittels auf die Zellzyklusposition der Zelle ist.

7. Verfahren zur Bestimmung der Wirkung eines Testmittels auf die Zellzyklusposition einer Säugerzelle, wobei das Verfahren umfasst:
a) Kultivieren einer stabilen Zelllinie nach Ansprüchen 1 - 4;
b) Bestimmen der Zellzyklusposition durch Überwachen von Signalen, die vom ersten und zweiten fluoreszierenden Protein gesendet werden;
c) Vergleichen der gesendeten Signale bei Vorhandensein des Testmittels mit einem bekannten Wert für die gesendeten Signale bei Nichtvorhandensein des Testmittels;
wobei ein Unterschied zwischen den gesendeten Signalen, die bei Vorhandensein des Testmittels gemessen werden, und dem bekannten Wert bei Nichtvorhandensein des Testmittels ein Indikator für die Wirkung des Testmittels auf die Zellzyklusposition der Zelle ist.

8. Verfahren zur Bestimmung der Wirkung des Säugerzellzyklus auf einen zellulären Prozess, der durch einen Prozessreporter überwacht wird, der bekanntermaßen in Reaktion auf ein Testmittel variiert, wobei das Verfahren umfasst:
a) Kultivieren einer stabilen Zelllinie nach Ansprüchen 1 - 4;
b) Bestimmen der Zellzyklusposition durch Überwachen von Signalen, die vom ersten und zweiten fluoreszierenden Protein gesendet werden; und
c) Überwachen der vom Prozessreporter gesendeten Signale, wobei der Prozessreporter vom ersten und zweiten fluoreszierenden Protein unterscheidbar ist,
wobei das Verhältnis zwischen durch Schritt b) bestimmter Zellzyklusposition und dem vom Prozessreporter gesendeten Signal ein Indikator dafür ist, ob der zelluläre Prozess zellzyklusabhängig ist oder nicht.

## Revendications

1. Lignée cellulaire stable exprimant :
i) une première chimère polypeptide comprenant une première protéine fluorescente liée sur un domaine de localisation subcellulaire dépendant de la phosphorylation de la zone de commande spécifique de terminaison C de l'Hélicase B (PSLD), l'emplacement de ladite chimère à l'intérieur d'une cellule mammifère est représentatif de la position du cycle cellulaire ; et
ii) une seconde chimère polypeptide comprenant une seconde protéine fluorescente liée à de la Cycline B1 de boîte D dans laquelle ladite seconde protéine fluorescente peut être détectée sur une cellule mammifère à une position prédéterminé sur le cycle cellulaire,
dans laquelle lesdites première et seconde protéines fluorescentes peuvent être distinguées l'une de l'autre et la lignée cellulaire stable peut être utilisée afin de déterminer la position de cycle cellulaire.

2. Lignée cellulaire stable selon la revendication 1, dans laquelle ladite protéine fluorescente est sélectionnée parmi la protéine fluorescente verte (GFP), la protéine fluorescente verte améliorée (EGFP), les protéines fluorescentes émeraude et Rouge J.

3. Lignée cellulaire stable selon l'une quelconque des revendications précédentes, dans laquelle la première protéine fluorescente est la protéine EGFP et la seconde protéine fluorescente est à fluorescente Rouge J, ou la première protéine fluorescente est à fluorescente Rouge J et la seconde protéine fluorescente est la protéine EGFP.

4. Lignée cellulaire stable selon l'une quelconque des revendications précédentes, dans laquelle la protéine fluorescente de la première chimère polypeptide est une protéine fluorescente rouge (RFP), et la seconde chimère polypeptide comprend 171 acides aminés de la terminaison amino de la cycline B1 couplés à une protéine fluorescente verte (GFP) exprimée sous la commande du promoteur de la cycline B1.

5. Procédé de détermination de la position de cycle cellulaire d'une cellule mammifère, ledit procédé comprenant :
a) la culture d'une lignée cellulaire stable selon les revendications 1 à 4 ; et
b) la détermination de la position de cycle cellulaire en surveillant les signaux émis par les première et seconde protéines fluorescentes.

6. Procédé de détermination de l'effet d'un agent de test sur la position de cycle cellulaire d'une cellule mammifère, ledit procédé comprenant :
a) la culture d'une lignée cellulaire stable selon les revendications 1 à 4 ; et
b) la détermination de la position de cycle cellulaire en surveillant les signaux émis par les première et seconde protéines fluorescentes dans lequel une différence entre les signaux émis mesurés en l'absence et en la présence dudit agent de test est représentative de l'effet de l'agent de test sur la position de cycle cellulaire de la cellule.

7. Procédé de détermination de l'effet d'un agent de test sur la position de cycle de cellulaire d'une cellule mammifère, ledit procédé comprenant :
a) la culture d'une lignée cellulaire stable selon les revendications 1 à 4 ;
b) la détermination de la position de cycle cellulaire en surveillant les signaux émis par les première et seconde protéines fluorescentes ;
c) la comparaison des signaux émis en la présence de l'agent de test avec une valeur connue pour des signaux émis en l'absence de l'agent de test ;
dans lequel une différence entre les signaux émis, mesurés en la présence de l'agent de test et ladite valeur connue en l'absence de l'agent de test est représentative de l'effet de l'agent de test sur la position de cycle cellulaire de la cellule.

8. Procédé de détermination de l'effet du cycle cellulaire de mammifère sur un processus cellulaire surveillé par un reporteur de processus qui est connu pour varier en réponse à un agent de test, ledit procédé comprenant :
a) la culture d'une lignée cellulaire stable selon les revendications 1 à 4 ;
b) la détermination de la position de cycle cellulaire en surveillant les signaux émis par les première et seconde protéines fluorescentes ; et
c) la surveillance des signaux émis par le reporteur de processus dans lequel le reporteur de processus peut être distingué des première et seconde protéines fluorescentes,
dans lequel la relation entre la position de cycle cellulaire déterminée par l'étape b) et le signal émis par le reporteur de processus est représentative du fait que le processus cellulaire dépend ou non du cycle cellulaire.
